(19) Europäisches Patentamt
European Patent Office
Office européen des brevets

(11) **EP 2 944 976 B1**

(12) **EUROPEAN PATENT SPECIFICATION**

(45) Date of publication and mention
of the grant of the patent:
**23.09.2020 Bulletin 2020/39**

(51) Int Cl.:
***G01S 7/52*** *(2006.01)*   ***G01S 15/89*** *(2006.01)*
***G10K 11/34*** *(2006.01)*

(21) Application number: **15167457.9**

(22) Date of filing: **13.05.2015**

(54) **BEAM FORMING APPARATUS, METHOD FOR FORMING BEAMS, ULTRASONIC IMAGING APPARATUS, AND ULTRASONIC PROBE**

STRAHLFORMUNGSVORRICHTUNG, VERFAHREN ZUR BILDUNG VON STRAHLEN, ULTRASCHALLABBILDUNGSVORRICHTUNG UND ULTRASCHALLSONDE

APPAREIL DE FORMATION DE FAISCEAU, PROCÉDÉ DE FORMATION DE FAISCEAUX, APPAREIL D'IMAGERIE PAR ULTRASON ET SONDE À ULTRASONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.05.2014 KR 20140057154**

(43) Date of publication of application:
**18.11.2015 Bulletin 2015/47**

(73) Proprietor: **Samsung Electronics Co., Ltd.**
**Gyeonggi-do 443-742 (KR)**

(72) Inventors:
• **KIM, Kyuhong**
  **Seoul (KR)**
• **KIM, Baehyung**
  **Gyeonggi-do (KR)**
• **PARK, Suhyun**
  **Gyeonggi-do (KR)**
• **SONG, Jong Keun**
  **Gyeonggi-do (KR)**

(74) Representative: **Grünecker Patent- und**
**Rechtsanwälte**
**PartG mbB**
**Leopoldstraße 4**
**80802 München (DE)**

(56) References cited:
**WO-A1-02/17296**     **WO-A1-2010/137453**
**WO-A1-2012/035723**  **US-A1- 2007 242 567**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 944 976 B1

## Description

BACKGROUND

### 1. Field

Exemplary embodiments relate to a beam forming apparatus, a method for forming beams, an ultrasonic imaging apparatus, and an ultrasonic probe.

### 2. Description of the Related Art

Beam forming is a process for combining electrical signals that are received through multiple channels from an ultrasonic imaging apparatus or radar apparatus to generate one signal.

A beam forming process may be used when an image is generated based on signals of a plurality of channels. For example, an ultrasonic imaging apparatus may acquire an ultrasonic image for a medical examination of an object by beam forming ultrasonic signals of a plurality of channels corresponding to received ultrasonic waves.

For instance, WO 2012/035723 A1 discloses ultrasound imaging with a two-stage beam former. In a first stage, signals from a line of transducer elements are beam formed by using a delay & sum beam former or an adaptive beam former. In the second stage, the signals output from the first stage are beam formed in the direction perpendicular to the line of transducer elements by using an adaptive beam former. Signals are acquired using a 2D transducer array or a mechanically scanned ID array.

WO 02/17296 A1 discloses a two-stage beam former for ultrasound imaging having an analog-to-digital converter in the first stage which may also perform multi-line beam forming.

WO 2010/137453 A1 discloses a beam former of an ultrasound diagnostic apparatus which comprises a data reducing section and a calculation section wherein the data reducing section combines signals of adjacent channels and wherein the calculation section uses the combined signals to calculate weights according to the DCMP (Directionally Constrained Minimization of Power) method and to form image data by weighting the combined signals using the calculated weights and by combining the weighted combined signals.

SUMMARY

One or more exemplary embodiments provide a beam forming apparatus, a method for forming beams, an ultrasonic imaging apparatus, and an ultrasonic probe that can combine a plurality of signals to acquire an image of good quality with minimum deterioration of image quality.

In accordance with a first aspect, the invention provides a beam forming apparatus as specified in claim 1.

In accordance with a second aspect, the invention provides a beam forming method as specified in claim 7.

In accordance with an aspect of an exemplary embodiment, an ultrasonic imaging apparatus includes an ultrasonic transducer configured to receive ultrasonic waves, convert the received ultrasonic waves into ultrasonic signals in a plurality of groups, the ultrasonic signals being electrical signals, and output the ultrasonic signals, and the beam forming apparatus configured to process the output ultrasonic signals.

In accordance with an aspect of an exemplary embodiment, an ultrasonic imaging apparatus includes an ultrasonic probe configured to receive ultrasonic waves, convert the received ultrasonic waves into ultrasonic signals in a plurality of groups, the ultrasonic signals being electrical signals, and output the ultrasonic signals and a main body configured to generate an ultrasonic image corresponding to the output ultrasonic signals, in which at least one of the ultrasonic probe and the main body includes the beam forming apparatus configured to process the output ultrasonic signals.

In accordance with an aspect of an exemplary embodiment, an ultrasonic probe includes an ultrasonic transducer configured to receive ultrasonic waves, convert the received ultrasonic waves into ultrasonic signals in a plurality of groups, the ultrasonic signals being electrical signals, and output the ultrasonic signals, and the beam forming apparatus configured to process the output ultrasonic signals.

BRIEF DESCRIPTION OF THE DRAWINGS

The above and/or other aspects will become more apparent by describing certain exemplary embodiments, with reference to the accompanying drawings, in which:

    FIG. 1 is a block diagram showing a beam forming apparatus according to an exemplary embodiment;
    FIG. 2 is a detailed block diagram showing a beam forming apparatus according to an exemplary embodiment;

FIG. 3 is a view for describing a beam forming apparatus according to an exemplary embodiment;

FIG. 4 is a view for describing a first beamformer of a beam forming apparatus;

FIG. 5 is a view for describing a time delay process according to an exemplary embodiment;

FIG. 6 is a view for describing a time delay process according to an exemplary embodiment;

FIG. 7 is a view for describing a first beamformer of a beam forming apparatus according to an embodiment of the invention;

FIG. 8 is a view for describing a second beamformer of a beam forming apparatus according to an exemplary embodiment;

FIG. 9 is a flowchart illustrating a method of forming a beam according to an exemplary embodiment;

FIG. 10 is a view showing an ultrasonic imaging apparatus according to an exemplary embodiment;

FIG. 11 is a block diagram showing an ultrasonic imaging apparatus according to an exemplary embodiment;

FIG. 12 is a view showing an ultrasonic probe according to an exemplary embodiment;

FIG. 13 shows an arrangement of transducers according to an exemplary embodiment;

FIG. 14 shows an arrangement of transducers according to an exemplary embodiment;

FIG. 15 is a block diagram showing an ultrasonic imaging apparatus according to an exemplary embodiment;

FIG. 16 is a block diagram showing an ultrasonic imaging apparatus according to an exemplary embodiment;

FIG. 17A is a flowchart illustrating a method according to an exemplary embodiment; and

FIG. 17B is a flowchart illustrating a method according to an exemplary embodiment.

## DETAILED DESCRIPTION

Certain exemplary embodiments are described in greater detail below with reference to the accompanying drawings.

In the following description, the same drawing reference numerals are used for the same elements even in different drawings. The matters defined in the description, such as detailed construction and elements, are provided to assist in a comprehensive understanding of exemplary embodiments. Thus, it is apparent that exemplary embodiments can be carried out without those specifically defined matters. Also, well-known functions or constructions are not described in detail since they would obscure exemplary embodiments with unnecessary detail.

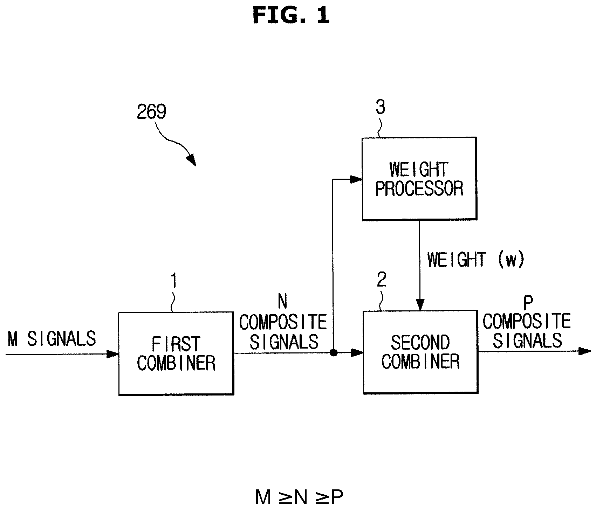

FIG. 1 is a block diagram showing a beam forming apparatus 269 according to an exemplary embodiment. As shown in FIG. 1, the beam forming apparatus may include a first combiner 1 configured to combine a plurality of input signals to output a plurality of first composite signals and a second combiner 2 configured to combine the plurality of first composite signals using weights (w) to output at least one second composite signal. The beam forming apparatus may further include a weight processor 3 configured to calculate the weights (w) used by the second combiner 2 based on the first composite signals. The first composite signal used in calculation of the weights (w) by the weight processor 3 may vary depending on an input signal or a composition process of the first combiner 1. Accordingly, the weights (w) calculated by the weight processor 3 may vary depending on M input signals or a composition process of the first combiner 1.

The number of input signals that are input to the first combiner 1 is M, the number of first composite signals that are combined by the first combiner 1 and then delivered to the second combiner 2 is N, and the number of second composite signals that are output by the second combiner 2 is P, in which each of M, N, and P may be a natural number. In this case, M may be greater than or equal to N, and N may be greater than or equal to P ($M \geq N \geq P$). In other words, the number of channels of a signal is reduced by the first combiner 1 and then reduced again by the second combiner 2. In some exemplary embodiments, the second combiner 2 may combine N composite signals to output one composite signal, i.e., P may be 1.

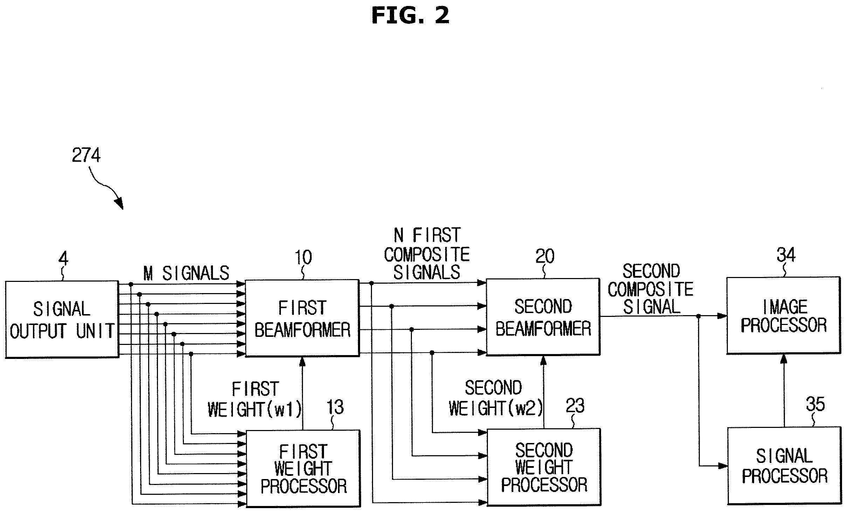

FIG. 2 is a detailed block diagram showing a beam forming apparatus 274 according to an exemplary embodiment. As shown in FIG. 2, the beam forming apparatus according to an exemplary embodiment may include a signal output unit 4, a first beamformer 10, a second beamformer 20, and a second weight processor 23. Further, in some exemplary embodiments, the beam forming apparatus may further include a first weight processor 13.

The signal output unit 4 may output signals of a plurality of channels. Each of the output signals may be an analog signal or digital signal. The signal output unit 4 may be an ultrasonic wave receiver such as an ultrasonic transducer that receives an ultrasonic wave and outputs an electrical signal corresponding to the received ultrasonic wave. Alternatively, the signal output unit 4 may be an electromagnetic wave receiver that receives an electromagnetic wave such as a microwave and outputs an electrical signal corresponding to the received electromagnetic wave. Signals of a plurality of channels may be delivered to the first beamformer 10. In some exemplary embodiments, the signals of the plurality of channels may be also delivered to the first weight processor 13.

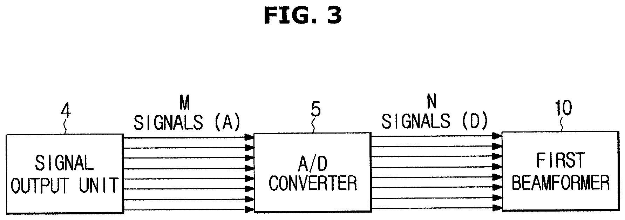

FIG. 3 is a view for describing a beam forming apparatus including an analog-to-digital (A/D) converter according to an exemplary embodiment. As shown in FIG. 3, according to an exemplary embodiment, an A/D converter 5 that converts an analog signal into a digital signal is provided between the signal output unit 4 and the first beamformer 10.

The A/D converter 5 may convert an analog signal output from the signal output unit 4 into a digital signal. Thus, the first beamformer 10 may process the digital signal instead of the analog signal output from the signal output unit 4. The A/D converter 5 may convert analog signals of M channels into digital signals of M channels. The A/D converter 5 may convert an analog signal into a digital signal using a sampling rate that is arbitrarily determined by a system designer or a user of the beam forming apparatus. In some exemplary embodiments, the A/D converter 5 may be omitted.

The signal of the plurality of channels output from the signal output unit 4 may be amplified by an amplifier and then delivered to the A/D converter 5 and/or the first beamformer 10. The amplifier for amplifying a magnitude of a voltage may adjust a signal delivered to the first beamformer 10 to a magnitude appropriate for processing and storing of the signal. A gain of the amplifier may be arbitrarily determined by a system designer or a user of the beam forming apparatus. In some exemplary embodiments, the amplifier may be omitted.

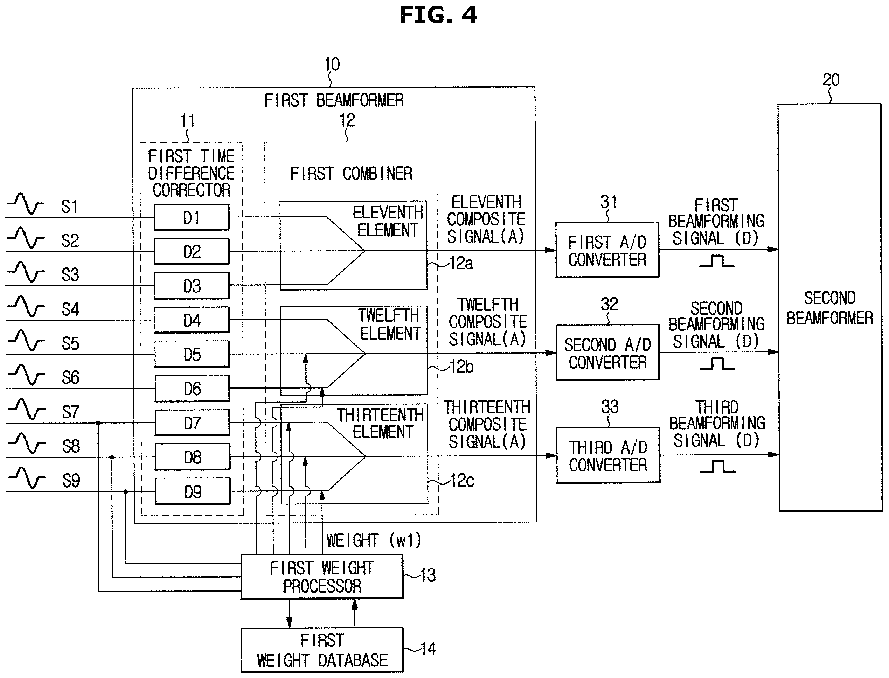

FIG. 4 is a view for describing a first beamformer of a beam forming apparatus. As shown in FIG. 4, the first beamformer 10 may include a first time difference corrector 11 and a first combiner 12. Input signals s1 to s9 of a plurality of channels which are input to the first beamformer 10 may be classified, i.e., grouped, into at least one group according to a predetermined criterion, for example, according to the first combiner 12 that combines the input signals s1 to s9 of the plurality of channels. For example, the signals of the plurality of channels may be classified into a plurality of groups as shown in FIG. 4. Specifically, first to third input signals s1, s2, and s3 that are combined by an eleventh element 12a are designated as a first group, fourth to sixth input signals s4, s5, and s6 that are combined by a twelfth element 12b are designated as a second group, and seventh to ninth input signals s7, s8, and s9 that are combined by a thirteenth element 12c are designated as a third group. According to the invention, at least one of the plurality of groups includes a signal of the same channel in common with a different group. For example, some of the signals input to the first beamformer 10 may be included in the first group and the second group. According to a comparative example, the plurality of groups may be disjoint, by only including input signals of different channels, respectively, as shown in FIG. 4. Signals in one group may have time differences corrected with respect to one another or may be combined by one of the eleventh to thirteenth elements 12a to 12c.

The first time difference corrector 11 may correct time differences between signals of the plurality of channels output from the signal output unit 4. Each of the signals of the plurality of channels may be an analog signal or digital signal. As shown in FIG. 4, the first time difference corrector 11 may include a plurality of first to ninth time difference correction elements D1 to D9 corresponding to the plurality of input channels. The first to ninth time difference correction elements D1 to D9 may be implemented by a predetermined semiconductor chip or circuit. The first to ninth time difference correction elements D1 to D9 may correct channel delays between signals of channels by delaying by a certain time (or not delaying) the signals of the channels corresponding to the first to ninth time difference correction elements D1 to D9.

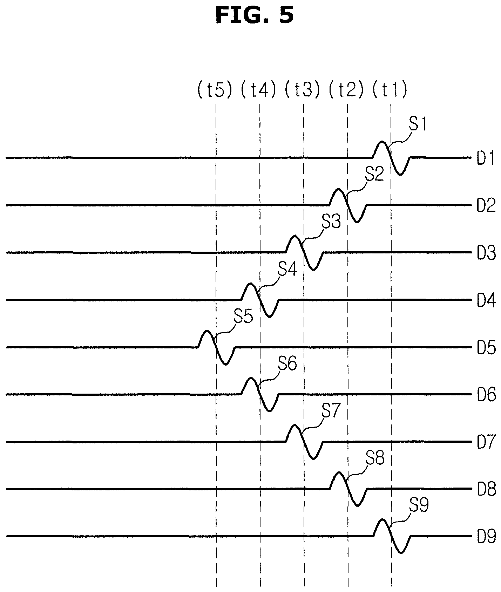

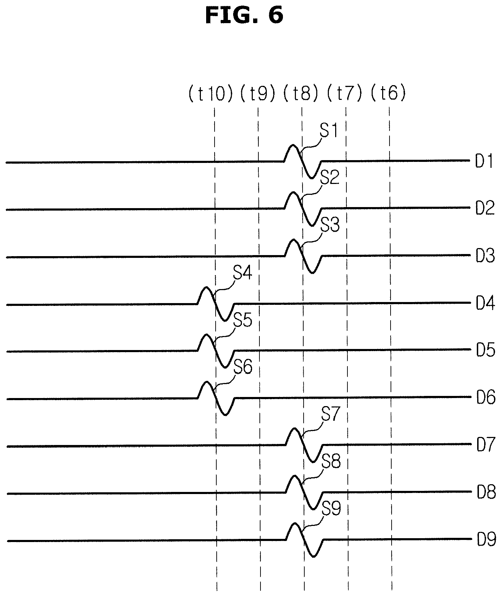

FIGS. 5 and 6 are views for describing a time delay process in a first beamformer according to an exemplary embodiment. According to an exemplary embodiment, as shown in FIGS. 5 and 6, each of the first to ninth time difference correction elements D1 to D9 may be set to correct time differences between signals in the same group. For example, as shown in FIG. 5, the first to third input signals s1 to s3 corresponding to the first group may be sequentially input to the first to third time difference correction elements D1, D2, and D3. The first to third input signals s1 to s3 are input to the first to third time difference correction elements D1 to D3 at times t1, t2, and t3, respectively, thus leading to time differences. As shown in FIG. 6, the first to third time difference correction elements D1 to D3 may correct time differences between the first to third input signals s1 to s3 by delaying the first input signal s1 and the second input signal s2, and output the first to third input signals s1 to s3 at the same time t8. Likewise, there may be a time difference between times t4 and t5 when the fourth to sixth input signals s4 to s6 in the second group are input to the fourth to sixth time difference correction elements D4, D5, and D6. As shown in FIG. 6, the fourth to sixth time difference correction elements D4 to D6 may correct time differences between the fourth to sixth input signals s4 to s6 by delaying the fourth input signal s4 and the sixth input signal s6, and output the fourth to sixth input signals s4 to s6 at the same time t10. Likewise, though the ninth to seventh input signals s9, s8, and s7 are input at different times t1 to t3, respectively, the seventh to ninth input signals s7 to s9 may be corrected by the seventh to ninth time difference correction elements D7, D8, and D9, respectively, and output at the same time t8.

The time differences between signals in the different groups might not be corrected while time differences between input signals (for example, s1 to s3) in the same group are corrected. For example, as shown in FIG. 6, the input signals s1 to s3 in the first group are output at the time t8 and the input signals s4 to s6 in the second group are output at the time t10. Thus, there is a time difference between both groups. Of course, there may be no time difference between some groups, such as between the first group and the third group.

The first combiner 12 may combine signals in which time differences have been corrected by the first time difference corrector 11, to generate, for example, eleventh to thirteenth composite signals. The time difference-corrected signals that are combined by the first combiner 12 may be analog signals or digital signals. As shown in FIG. 4, the first combiner 12 may include a plurality of elements, for example, the eleventh to thirteenth elements 12a to 12c. Each of the elements 12a to 12c may combine signals of some of the plurality of channels. As shown in FIG. 4, each of the elements 12a to 12c may combine signals in groups corresponding to the elements 12a to 12c to output a plurality of first composite signals, respectively. For example, the eleventh element 12a may combine input signals s1 to s3 in a first group to output an eleventh composite signal, and the twelfth element 12b may combine input signals s4 to s6 in a second group to output a twelfth composite signal. Furthermore, the thirteenth element 12c may combine input signals s7 to s9 in a third group to output a thirteenth composite signal.

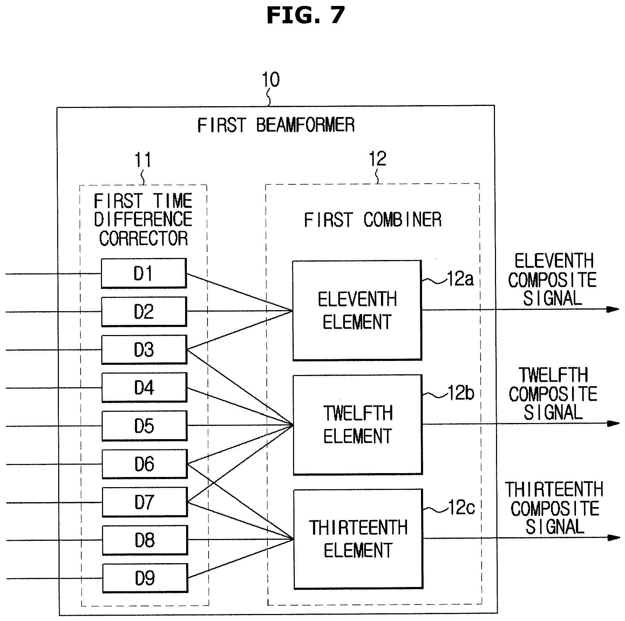

FIG. 7 is a view for describing a first beamformer of a beam forming apparatus according to an embodiment of the invention. At least one of the eleventh to thirteenth elements 12a to 12c of the first combiner 12 performs a combination using the same signal. For example, at least one of the signals in a group that are combined by the twelfth element 12b is the same as at least one of the signals in the group that are combined by one of the eleventh and thirteenth elements 12a and 12c.

As shown in FIG. 7, the eleventh element 12a may combine signals in which time differences have been corrected by the first to third time difference correction elements D1 to D3 to generate an eleventh composite signal, and the twelfth element 12b may combine the signal in which the time difference has been corrected by the third time difference correction element D3, which is used for the combination of the eleventh element 12a, with signals in which time differences have been corrected by the fourth to seventh time difference correction elements D4 to D7 to generate a twelfth composite signal. The twelfth element 12b may combine signals in which time differences have been corrected by the third to seventh time difference correction elements D3 to D7 to generate a twelfth composite signal, and the thirteenth element 12c may combine the signals in which time differences have been corrected by the sixth and seventh time difference correction elements D6 and D7, with signals in which time differences have been corrected by eighth and ninth time difference correction elements D8 and D9 to generate a thirteenth composite signal.

Each of time difference correction elements D1 to D9 may delay signals to correct time difference between the signals such that a plurality of elements, for example, the eleventh and twelfth elements 12a and 12b may combine appropriate signals. Time differences of signals in different groups, for example, the first group and the second group, may be corrected by the time difference correction elements D1 to D7 such that all of the signals of all of the groups or some of the signals of some of the groups may be output at the same time.

At least one of the eleventh to thirteenth elements 12a to 12c may combine the input signals s1 to s9 while applying a first weight w1. The first weight w1 may be added to at least one of the input signals s1 to s9 in each of the groups, and be used to strengthen, weaken, or remove a specific signal. According to an exemplary embodiment, all or some of the eleventh to thirteenth elements 12a to 12c may combine the input signals s1 to s9 in each of the groups using the first weight w1. For example, only the thirteenth element 12c may combine the input signals s7 to s9 using the first weight w1. The first weight w1 may be predefined irrespective of the input signals s1 to s9 or may be varied depending on at least one of the input signals s1 to s9.

The first weight processor 13 may determine a first weight w1 to deliver the determined first weight w1 to the first combiner 12. The first weight processor 13 may search a first weight database 14 to read a predetermined weight w1 and deliver the weight w1 to the first combiner 12. In addition, the first weight processor 13 may receive at least one of the input signals s1 to s9, read a predetermined weight calculation method from the first weight database 14, and then determine the first weight w1 based on the input signal and the weight calculation method. As shown in FIG. 4, the first weight processor 13 may deliver the weight to only some of the eleventh to thirteenth elements 12a to 12c. In some exemplary embodiments, the first weight processor 13 or the first weight database 14 may be omitted.

As described above, the first beamformer 10 may output a plurality of first composite signals based on input signals s1 to s9 of a plurality of channels. A beam forming process performed by the first beamformer 10 may be expressed as Equation 1 below:

[Equation 1]

$$z_{jk}(t) = \Sigma_{i=1}^{K_j} \; a_i \, x_i \left( t - \Delta_{ik}(t) \right).$$

In Equation 1, i and j are positive integers, $z_{jk}(t)$ is a first composite signal of a jth group, i is an index indicating a channel of each signal in the jth group, k is an index for each beam to be formed, and $K_j$ is the number of channels of input signals in the jth group. According to the comparative example disclosed in figure 4, $K_j$ may satisfy Equation 2 below:

[Equation 2]

$$M = \Sigma_{j=1}^{J} \; K_j.$$

In Equation 2, M is the total number of channels that are finally combined through the first and second beamformers 10 and 20 and J is the number of groups.

In Equation 1, $a_i$ is a first weight added to each channel, $\Delta_{ik}(t)$ is a time difference correction value that is used for the first time difference corrector 11 to correct a time difference, and $x_i(t-\Delta_{ik}(t))$ is a signal in which a time difference has been corrected by the first time difference corrector 11.

A signal output from the first combiner 12 may be directly delivered to the second beamformer 20. In some exemplary embodiments, the signal output from the first combiner 12 may be delivered the second beamformer 20 via first, second, and third A/D converters 31, 32, and 33.

According to an exemplary embodiment, as shown in FIG. 4, the beam forming apparatus may further include the first, second, and third A/D converters 31 to 33 that convert first composite signals, which are analog signals output from the first beamformer 10, for example, eleventh to thirteenth composite signals into digital signals, respectively. The first, second, and third A/D converters 31 to 33 convert the eleventh to thirteenth composite signals into digital signals, respectively, such that the second beamformer 20 may process the eleventh to thirteenth composite signals in a digital form. Each of the first, second, and third A/D converters 31 to 33 may convert an analog signal into a digital signal using a sampling rate that is arbitrarily determined by a system designer or a user of the beam forming apparatus.

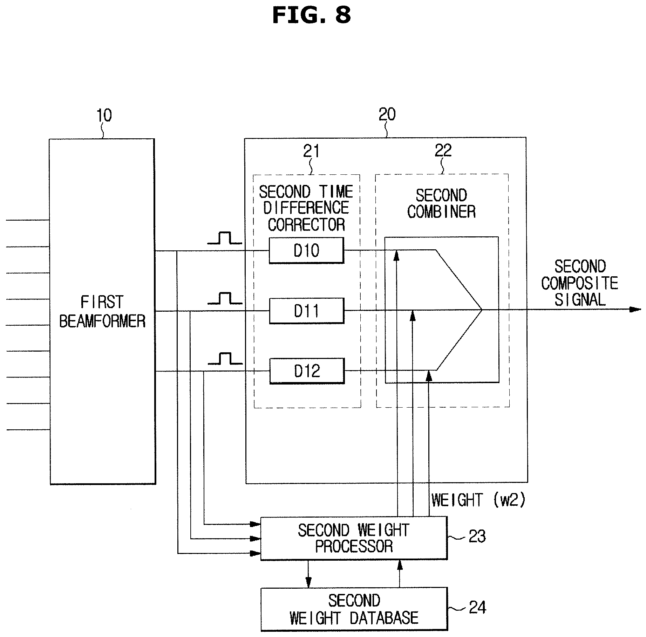

FIG. 8 is a view for describing a second beamformer of a beam forming apparatus according to an exemplary embodiment. As shown in FIG. 8, the second beamformer 20 may include a second time difference corrector 21 and a second combiner 22.

The second time difference corrector 21 may correct time differences between first composite signals. As shown in FIG. 6, there may be a time difference between signals in different groups. Since there is a time difference between signals in different groups, there may be a time difference between first composite signals acquired by combining the groups. As such, the second time difference corrector 21 may correct a time difference between first composite signals and deliver the first composite signals in which the time difference has been corrected to the second combiner 22.

The second combiner 22 may combine a plurality of first composite signals in which time differences have been corrected to generate at least one second composite signal. The second combiner 22 may output one second composite signal or output a plurality of second composite signals.

According to an exemplary embodiment, as shown in FIGS. 2 and 8, the second combiner 22 may combine first composite signals using a second weight w2 to generate a second composite signal. The second weight w2 may be added to at least one of the first composite signals and be used to strengthen, weaken, or remove a specific first composite signal. The second weight w2 may be determined according to the first composite signal, by a second weight processor 23. As shown in FIGS. 2 and 8, the second weight processor 23 may receive the first composite signal directly from the first beamformer 10, and determine the second weight w2 based on the first composite signal. Accordingly, the second weight w2 may be varied depending on the first composite signal. The second weight processor 23 may deliver the determined second weight w2 to the second combiner 22.

According to an exemplary embodiment, the second weight processor 23 may detect a weight appropriate for the first composite signal from a second weight database 24 based on the first composite signal and then designate the detected weight as the second weight w2. According to an exemplary embodiment, the second weight processor 23 may read a predetermined weight calculation method from the second weight database 24 and then determine the second weight w2 using the delivered first composite signal and the weight calculation method. For example, the second weight processor 23 may apply weights to the first composite signals and calculate a sum of absolute values of the first composite signals having the applied weights or a sum of absolute values of squares of the first composite signals having the applied weights. Next, the second weight processor 23 may detect weights that may minimize the sum of absolute values of the first composite signals having the applied weights or the sum of absolute values of squares of the first composite signals having the applied weights based on a result of the calculation, determine the weights as second weights w2, and deliver the second weights w2 to the second combiner 22.

The above-described beam forming process performed by the second beamformer 20 may be expressed as Equation 3 below:

[Equation 3]

$$y_k(t) = \Sigma_{j=1}^{N} \, b_j z_{jk} \left( t - \Delta_{jk}(t) \right)$$

.

In Equation 3, $y_k(t)$ is a second composite signal, j is an index for identifying a first composite signal, and N is the number of first composite signals input to the second beamformer 20. Since the number of first composite signals is equal to the number of signal groups, N may have the same value as J of Equation 1.

In Equation 3, $b_j$ is a second weight added to each first composite signal, $\Delta_{jk}(t)$ is a time difference correction value that is used for the second time difference corrector 21 to correct a time difference, and $z_j(t-\Delta_{jk}(t))$ is a first composite signal in which a time difference has been corrected by the second time difference corrector 21.

As a result, according to Equations 1 and 3, the second composite signal may be calculated according to Equation 4 below:

[Equation 4]

$$y_k(t) = \sum_{j=1}^{N} b_j \sum_{i=1}^{K_j} a_i x_i \left( t - \Delta_{ijk}(t) \right)$$

In equation 4, $\Delta_{ijk}(t)$ is a time difference correction value at a time when an input signal has passed through both the first beamformer 10 and the second beamformer 20.

According to an exemplary embodiment, as shown in FIG. 2, the second composite signal may be delivered to an image processor 34 and/or signal processor 35.

The image processor 34 may generate an image corresponding to the second composite signal based on the second composite signal. The signal processor 35 may perform various signal processing, such as filtering, demodulation, and compression, on the second composite signal and deliver the signal on which the signal processing has been performed to the image processor 34. The image processor 34 may generate an image corresponding to the signal on which the signal processing has been performed based on the signal on which the signal processing has been performed.

If the second combiner 22 of the beamformer 20 outputs a plurality of second composite signals, the beam forming apparatus may further include a third beamformer (not shown) for combining the plurality of second composite signals. The third beamformer may output a third composite signal by combining the plurality of second composite signals through the same procedure as that of the first beamformer 10 or second beamformer 20.

The above-described beam forming apparatus may be implemented with a semiconductor chip that may perform a calculation or storage function and a printed circuit board having the semiconductor chip disposed thereon. In addition, the above-described beam forming apparatus may be applied to various devices that use beam forming, as for example, an ultrasonic imaging apparatus, a radar apparatus, and/or an antenna.

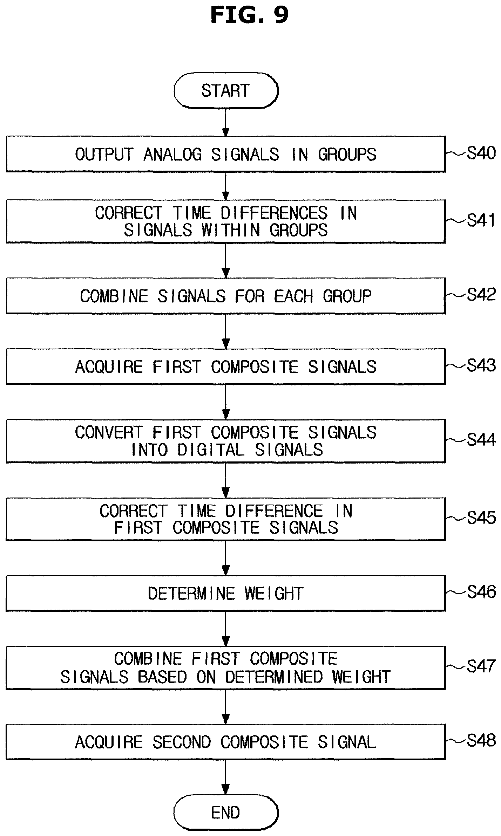

A method of forming a beam according to an exemplary embodiment is described in detail below with reference to FIG. 9.

As shown in FIG. 9, in the method of forming a beam according to an exemplary embodiment, signals in a plurality of groups may be output in operation s40. Each group may include a plurality of signals. The output signals may be analog signals.

The first beamformer 10 may correct time differences between signals for each group in operation s41. When the time differences between signals for each group are corrected, the first beamformer 10 may combine signals in operation s42. The combination of the signals may be performed for each group. In some exemplary embodiments, a predetermined weight may be further added when combining the signals. The predetermined weight may be acquired based on signals in each group or may be defined irrespective of the signals in each group. As a result, a plurality of first composite signals corresponding to respective groups may be acquired in operation s43. When signals in the group are analog signals, the first composite signals may also be analog signals. The first, second, and third A/D converters 31 to 33 may convert first composite signals which are analog signals, into a plurality of first composite signals which are digital signals in operation s44.

The second beamformer 20 may receive the digital-converted plurality of first composite signals and correct time differences between the received digital-converted plurality of first composite signals in operation s45. The second weight processor 23 may determine a weight to be used in the second beamformer 20 using the digital-converted plurality of first composite signals in operation s46. The second beamformer 20 may apply the determined weight to the combination of the digital-converted plurality of first composite signals in operation s47 to acquire at least one second composite signal in operation s48. The second composite signal may be delivered to the image processor 34, the signal processor 35, or the third beamformer.

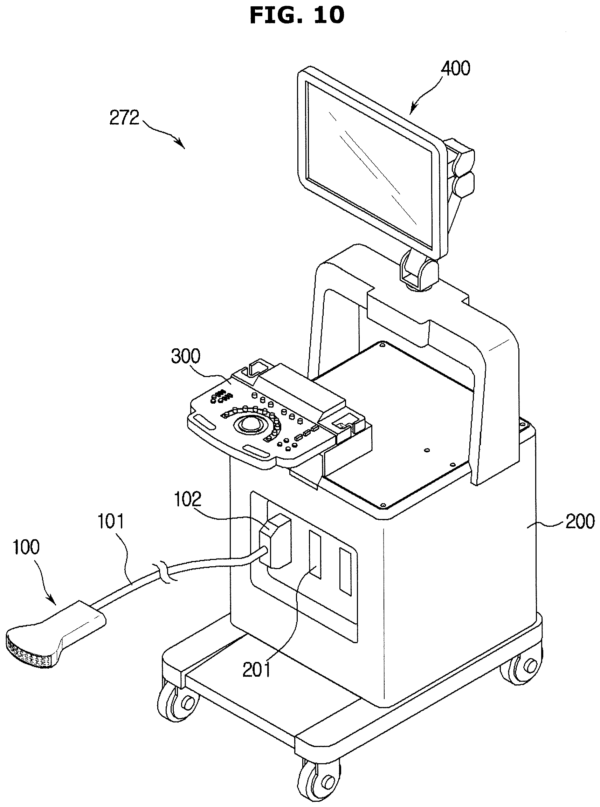

An ultrasonic imaging apparatus according to an exemplary embodiment, which includes the beam forming apparatus described above with reference to FIGS. 1-9, is described in detail below with reference to FIGS. 10 to 16, and the details described above with reference to FIGS. 1-9 are omitted.

The ultrasonic imaging apparatus collects an ultrasonic wave delivered from inside an object and then acquires a tomography image of a variety of tissues or structures inside the object, for example, a tomography image of a soft tissue or an image of blood flow. An ultrasonic imaging apparatus that transmits an ultrasonic wave into the object, receives a reflected ultrasonic wave, and generates an ultrasonic image, is described below as an example of an ultrasonic imaging apparatus including the beam forming apparatus. However, the ultrasonic imaging apparatus including the beam forming apparatus is not limited to the below-described ultrasonic imaging apparatus. For example, the ultrasonic imaging apparatus including the beam forming apparatus may be a photoacoustic imaging apparatus, a vibroacoustography, or picosecond ultrasonics. In addition, the ultrasonic imaging apparatus including the beam forming apparatus may include various apparatuses that receive an ultrasonic wave and generate an ultrasonic image.

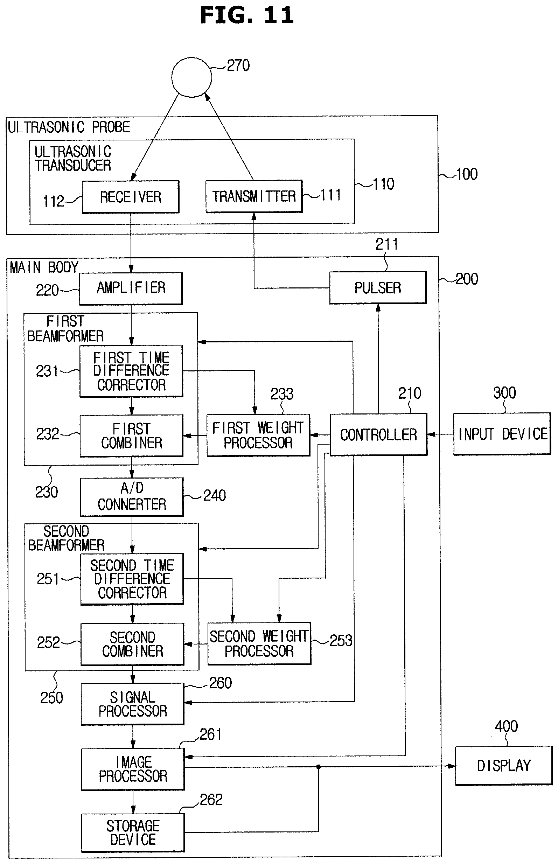

As shown in FIGS. 10 and 11, the ultrasonic imaging apparatus 272 may include an ultrasonic probe 100, a main body 200, an input device 300, and a display 400. The ultrasonic probe 100, the main body 200, the input device 300, and the display 400 may deliver data in a wired or wireless manner. For example, as shown in FIG. 10, the ultrasonic probe 100 and the main body 200 are connected through a connection cable 101 having one end connected to the ultrasonic probe 100 and the other end provided with a connector 102 that can be coupled to or separated from a slot 201 of the main body 200, and are configured to transmit and receive commands or data therebetween using the connection cable 101. According to an exemplary embodiment, the input device 300 and the display 400 may be directly disposed on the main body 200, as shown in FIG. 10. According to another exemplary embodiment, at least one of the input device 300 and the display 400 may be provided to be spaced apart from the main body 200. For example, the input device 300 or the display 400 may be disposed on a workstation (not shown) that is provided separately from the main body 200.

The ultrasonic probe 100 may receive an ultrasonic wave generated inside an object and convert the received ultrasonic wave into an ultrasonic signal which is an electrical signal. The ultrasonic probe 100 may generate an ultrasonic wave and then transmit the generated ultrasonic wave to a targeted part inside an object 270. According to an exemplary embodiment, the ultrasonic probe 100 may generate and transmit an ultrasonic wave of a predetermined frequency based on a control signal of a controller 210 of the main body 200. According to an exemplary embodiment, the ultrasonic probe 100 may generate and transmit the ultrasonic wave of the predetermined frequency based on a control signal of the controller 210 provided inside a housing of the ultrasonic probe 100.

The ultrasonic probe 100 may include at least one of a linear array probe, a convex array probe, a sector phased array probe, and a mechanical sector array probe.

As shown in FIG. 11, the ultrasonic probe 100 may include an ultrasonic wave transmitter 111 and an ultrasonic wave receiver 112. The ultrasonic wave transmitter 111 may generate an ultrasonic wave of a predetermined frequency according to an electrical voltage that is delivered from a pulser 211, and transmit the generated ultrasonic wave to a target of the object 270. The ultrasonic wave receiver 112 may receive an echo ultrasonic wave that is reflected by the target or generated according to a laser transmitted onto the target and generate an ultrasonic signal, which is an electrical signal corresponding to the echo ultrasonic wave. In some exemplary embodiments, the ultrasonic probe 100 may include an ultrasonic wave transceiver (not shown) that may perform ultrasonic transmission and reception functions. When the ultrasonic probe 100 includes the ultrasonic wave transceiver, the ultrasonic probe 100 may further include a switch for switching between transmission and reception. The ultrasonic wave receiver 112 and the ultrasonic wave transceiver may output ultrasonic signals of a plurality of channels. The ultrasonic wave transmitter 111, the ultrasonic wave receiver 112, and the ultrasonic wave transceiver may include an ultrasonic transducer 110a.

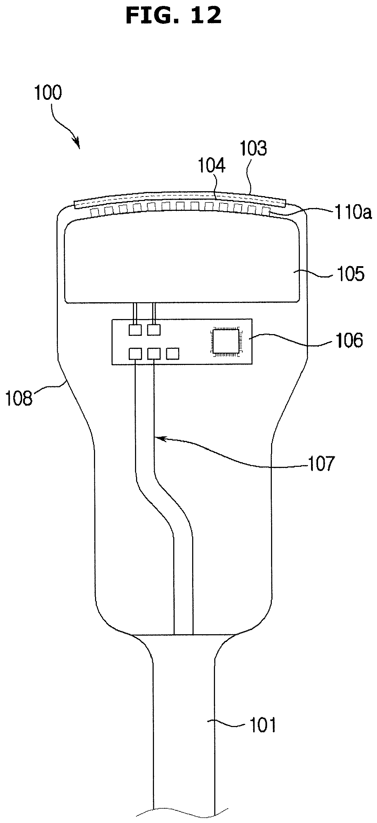

FIG. 12 is a view showing an ultrasonic probe according to an exemplary embodiment. The ultrasonic probe 100 may include an acoustic lens 103, an acoustic matching layer 104, the ultrasonic transducer 110a, an ultrasonic transducer support 105, an ultrasonic probe processor 106, and an electrical lead 107.

The acoustic lens 103 may be designed to focus and defocus an acoustic wave or ultrasonic wave. According to an exemplary embodiment, the acoustic lens 103 may refract an ultrasonic wave generated by the ultrasonic transducer 110a such that the ultrasonic wave may be focused on the target.

The acoustic matching layer 104 may provide a function of holding straightness, a waveform characteristic, and strength of the ultrasonic wave generated by the ultrasonic transducer 110a or minimizing reflection of the ultrasonic wave by another medium.

The ultrasonic transducer 110a may generate an ultrasonic wave of a predetermined frequency by converting an electrical signal of the predetermined frequency into a mechanical vibration of the same frequency. Specifically, when a voltage generated by the pulser 211 is applied to the ultrasonic transducer 110a, a piezoelectric vibrator or thin film of the ultrasonic transducer 110a may be vibrated and thus the ultrasonic transducer 110a may generate a plurality of ultrasonic waves according to the vibration of the piezoelectric vibrator or thin film. The ultrasonic wave generated by the ultrasonic transducer 110a may be transmitted into the object 270 and focused on a target inside the object 270. The ultrasonic wave may be focused on one target (single focusing) or a plurality of targets (multi-focusing).

The ultrasonic transducer 110a may receive an ultrasonic wave, vibrate at a frequency corresponding to a frequency of the received ultrasonic wave according to the received ultrasonic wave, and output an ultrasonic signal which is an electrical signal. Since one ultrasonic transducer 110a may output a signal of one channel, a plurality of ultrasonic transducers 110a may be needed to output signals of a plurality of channels. The output ultrasonic signals may be delivered to an amplifier 220, first beamformer 230, or second beamformer 250.

The ultrasonic transducer 110a may include a piezoelectric ultrasonic transducer using a piezoelectric effect of a piezoelectric material, a magnetostrictive ultrasonic transducer using a magnetostriction effect of a magnetic material, or a capacitive micromachined ultrasonic transducers (cMUT) using vibrations of hundreds or thousands of micro-processed thin films. In addition, the ultrasonic transducer 110a may include various other types of transducers that may generate an ultrasonic wave according to an electrical signal or generate an electrical signal according to the ultrasonic wave.

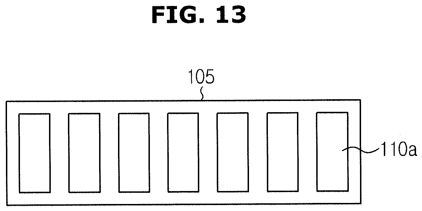

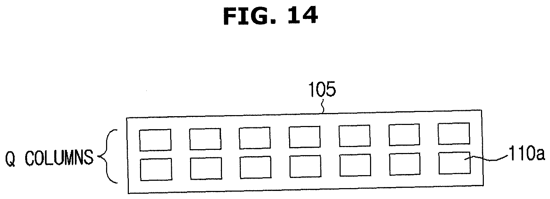

The ultrasonic transducers 110a may be disposed on one surface of the ultrasonic transducer support 105. The ultrasonic transducers 110a may be arranged in various forms. FIGS. 13 and 14 are views showing examples of arrangement of ultrasonic transducers. As shown in FIG. 13, the ultrasonic transducers 110a may be arranged in a straight line on one surface of the ultrasonic transducer support 105. Further, as shown in FIG. 14, the ultrasonic transducers 110a may be arranged in a plurality of columns Q. According to an exemplary embodiment, the ultrasonic transducer 110a may be arranged in two columns on one surface of the ultrasonic transducer support 105 (two-dimensional array). However, a number of columns is not limited to two. When the ultrasonic transducers 110a are arranged in a plurality of columns, a high resolution in a vertical direction may be maintained.

The ultrasonic transducer support 105 may absorb an ultrasonic wave transmitted in an opposite direction among ultrasonic waves generated by the ultrasonic transducer 110a or emit heat generated during an operation of the ultrasonic transducer 110a while supporting the ultrasonic transducer 110a.

The ultrasonic probe processor 106 may perform various processing on the ultrasonic signal. For example, the ultrasonic probe processor 106 may amplify the ultrasonic signal delivered by the ultrasonic transducer 110a or convert the ultrasonic signal in an analog form into a digital signal. The ultrasonic probe processor 106 may generate a control signal for generating an overall operation of the ultrasonic probe 100. The ultrasonic probe processor 106 may include a variety of semiconductor chips and a printed circuit board. The semiconductor chips may include a memory semiconductor or a non-memory semiconductor. The ultrasonic probe processor 106 may be disposed on a rear surface or side surface of the ultrasonic transducer support 105. Alternatively, the ultrasonic probe processor 106 may be disposed at any position inside a housing 108 of the ultrasonic probe 100.

The electrical lead 107 may deliver an ultrasonic signal or a beam-formed ultrasonic signal to the main body 200. The electrical lead 107 may be a portion of the connection cable 101. The ultrasonic signal may be delivered to the main body 200 through the electrical lead 107, the connector 102, and the slot 201. In some exemplary embodiments, the beam-formed ultrasonic signal may be delivered from the ultrasonic probe 100 to the main body 200 through the electrical lead 107, the connector 102, and the slot 201.

As shown in FIG. 11, the main body 200 may include the controller 210, the pulser 211, the amplifier 220, a first beamformer 230, an A/D converter 240, a second beamformer 250, a signal processor 260, an image processor 261, and a storage device 262. Some of the above components may be omitted. Further, at least one of the controller 210, the pulser 211, the amplifier 220, the first beamformer 230, the A/D converter 240, the second beamformer 250, the signal processor 260, the image processor 261, and the storage device 262 may be provided in the ultrasonic probe 100 or provided in a workstation connected to the main body 200 over a wired and/or wireless communication network.

The controller 210, the pulser 211, the amplifier 220, the first beamformer 230, the A/D converter 240, the second beamformer 250, the signal processor 260, and the image processor 261 may include at least one of a central processing unit (CPU) and a graphic processing unit (GPU), which are provided within the main body 200 or workstation. The central processing unit and the graphic processing unit may be implemented using various semiconductor chips and a printed circuit board having the semiconductor chips disposed thereon. The storage device 262 may include a semiconductor memory device, a magnetic disk memory device, or an optical disk memory device disposed inside or outside the main body 200 or workstation. When a command or data is delivered from one component provided in the main body 200 to another component 210 to 262, the command or data may be temporarily or non-temporarily stored in a memory for convenience in calculation processing. The memory may be a volatile memory device or non-volatile memory device.

The controller 210 may control an operation of the ultrasonic imaging apparatus 272 according to a user's command or a predefined setting. The user's command may be input through the input device 300. The predefined setting may be stored in a storage device 262. According to an exemplary embodiment, the controller 210 may generate a control command according to a frequency of an ultrasonic wave to be transmitted and deliver the generated control command to the pulser 211. The control command may include information on a frequency or magnitude of a voltage to be applied to the ultrasonic wave transmitter 111.

The pulser 211 may generate a voltage for driving the ultrasonic transducer 110a of the ultrasonic wave transmitter 111. The pulser 211 may generate a voltage of a predetermined amplitude and a predetermined frequency according to a control command received from the controller 210. A frequency and a strength of the ultrasonic wave generated by the ultrasonic transducer 110a may depend on the amplitude and frequency of the voltage generated by the pulser 211. The voltage output by the pulser 211 may be applied to ultrasonic transducers 110a of the ultrasonic wave transmitter 111 at different times having a certain time difference such that the transmitted ultrasonic wave may be focused or steered.

The amplifier 220 may amplify voltages of ultrasonic signals of a plurality of channels which are output by the ultrasonic wave receiver 112 to scale the ultrasonic signals to an appropriate magnitude for processing of the first beamformer 230. A gain of the amplifier 220 may be arbitrarily determined by a system designer or a user. The amplifier 220 may be omitted. An ultrasonic signal amplified by the amplifier 220 may be delivered to the first beamformer 230. In some exemplary embodiments, the ultrasonic signal amplified by the amplifier 220 may be delivered to the first beamformer 230 through an A/D converter 10 (shown in FIG. 2) that converts an ultrasonic signal from an analog signal to a digital signal.

Ultrasonic signals of a plurality of channels that are output by the ultrasonic wave receiver 112 or amplified by the amplifier 220 may be focused through two stages by the first beamformer 230 and the second beamformer 250, as described in detail above. Therefore, the detailed repeated descriptions are omitted.

The first beamformer 230 may focus ultrasonic signals for each group to generate a plurality of first composite signals. The first beamformer 230 may include a first time difference corrector 231 and the first combiner 232.

The first time difference corrector 231 may correct time differences between ultrasonic signals of a plurality of channels, as described above with reference to FIGS. 5 and 6.

The first combiner 232 may combine ultrasonic signals corresponding to the same group among the ultrasonic signals in which time differences have been corrected by the first time difference corrector 231 to generate a plurality of first composite signals, as described above with reference to FIGS. 4 and 7. As shown in FIG. 11, the first combiner 232 may combine the ultrasonic signals of the plurality of channels using a first weight that is delivered by a first weight processor 233. The first weight may be predefined irrespective of the ultrasonic signal or may be determined according to the ultrasonic signal.

A beam forming process performed by the first beamformer 230 may be expressed as Equation 1 above.

The A/D converter 240 may convert a first composite signal output by the first beamformer 230 into a digital signal.

The second beamformer 250 may combine a plurality of first composite signals to generate a second composite signal. The second beamformer 250 may include a second time difference corrector 251 and a second combiner 252.

The second time difference corrector 251 may correct time differences between first composite signals output by the first beamformer 230. The first composite signals in which the time differences have been corrected may be delivered to the second combiner 252.

The second combiner 252 may combine a plurality of first composite signals in which time differences have been corrected to generate a second composite signal. The second combiner 252 may output one second composite signal or output a plurality of second composite signals. The second combiner 252 may combine first composite signals using a second weight which may be determined by a second weight processor 253.

A beam forming process performed by the second beamformer 250 may be expressed as Equation 3 above.

As shown in FIG. 14, when the ultrasonic transducers 110a are arranged in a plurality of columns, complexity of the ultrasonic imaging apparatus may increase depending on the number of ultrasonic transducers 110a. In this case, if a beamformer samples an ultrasonic signal output by the ultrasonic transducer 110a with a first frequency $4f_0$, which is four times a central frequency $f_0$, a clarity of the ultrasonic image may be deteriorated due to degradation of a delay resolution of the beamformer. If the beamformer samples the ultrasonic signal with a second frequency $16f_0$, which is sixteen times the central frequency $f_0$, the clarity of the ultrasonic image may be improved, but an amount of data to be processed may increase. When the amount of data increases, the complexity of the ultrasonic imaging apparatus may also increase correspondingly. Furthermore, even if the beamformer samples the ultrasonic signal with the first frequency $4f_0$, an interpolation device is further needed to improve an image quality using an interpolation method.

If the second beamformer 250 combines first composite signals using a second weight that varies depending on the first composite signals as described above, the sampling frequency of the first beamformer 230 may be reduced to four times the central frequency, that is $4f_0$, and there is no need to design the interpolation device to increase accuracy of time difference correction. Specifically, when the variable second weight is used according to the first composite signal, even when the sampling is performed with the first frequency $4f_0$ which is four times the central frequency $f_0$, sidelobe energy that determines the clarity of the ultrasonic image may be kept low, and a bandwidth of the ultrasonic signal that determines resolution of the ultrasonic image may be reduced. Accordingly, a quality of the ultrasonic image to be acquired is enhanced. Therefore, an image quality may be prevented from being degraded due to low delay resolution even when the time difference is corrected in an analog manner, and thus acquiring an ultrasonic image having resolution of a case in which the sampling frequency is the second frequency $16f_0$ which is sixteen times the central frequency $f_0$ without increasing the complexity of the apparatus is possible although the sampling frequency of the ultrasonic image is actually the first frequency $4f_0$. In addition, the sampling frequency of the first beamformer 230 may be reduced to four times $(4f_0)$ the central frequency $f_0$, thereby using a memory device having a smaller capacity in the first beamformer 230. Accordingly, cost-effectiveness may be enhanced when the ultrasonic imaging apparatus is manufactured.

The second composite signal output by the second beamformer 250 may be delivered to at least one of the signal processor 260 and the image processor 261.

The signal processor 260 may perform various signal processing on the second composite signal. For example, the signal processor 260 may perform a filtering process for applying a filter to the second composite signal to remove signals other than signals in a specific bandwidth. In addition, the signal processor 260 may convert a voltage of the ultrasonic signal from a wireless frequency form to a video signal form. In addition, the signal processor 260 may perform a compression process for reducing an amplitude difference between the ultrasonic signals. The signal processor 260 may be omitted.

The image processor 261 may convert the second composite signal or the signal in which signal processing has been performed by the signal processor 260 into an image. The image processor 261 may generate an ultrasonic image using scan conversion. The ultrasonic image may be an amplitude mode (A-mode), brightness mode (B-mode), or motion mode (M-mode) ultrasonic image. The A-mode ultrasonic image is an ultrasonic image obtained by imaging a reflection strength using amplitude based on a distance between a target and the ultrasonic probe 100 or a time taken for the distance. The B-mode ultrasonic image is an ultrasonic image obtained by imaging a strength of the ultrasonic wave using brightness. The M-mode ultrasonic image is an ultrasonic image obtained by imaging a degree of change in an operation of an object. The ultrasonic image may include a Doppler image using a Doppler effect. The image processor 261 may correct an ultrasonic image by correcting brightness, luminance, sharpness, contrast, and/or color in an entire or a portion of the ultrasonic image such that a user may clearly view a tissue in the ultrasonic image. Furthermore, the image processor 261 may remove noise or interpolate a pixel. In addition, the image processor 261 may generate a panorama image, and generate a three-dimensional ultrasonic image using volume data. The image processing may be performed according to a user's command or a predefined setting.

The image processor 261 may store the generated or corrected ultrasonic image in the storage device 262 or display the ultrasonic image on the display 400.

The storage device 262 may temporarily or non-temporarily store an ultrasonic image generated by the image processor 261. The storage device 262 may temporarily or non-temporarily store a first composite signal, a second composite signal, or a second composite signal that has been processed by the signal processor 260. An ultrasonic image stored in the storage device 262 may be displayed on the display 400 or transmitted to another storage device (not shown) according to a user's requirement that is input through the input device 300 or an instruction of the controller 210. The ultrasonic image stored in the storage device 262 may be delivered to a workstation that is separately connected to the ultrasonic imaging apparatus 272. Furthermore, the ultrasonic image stored in the storage device 262 may be sent to an external server device or the like through a wired or wireless network. The server device may send the received ultrasonic image or the like to a separate terminal, for example, a desktop computer, a smartphone, a cellular phone, a tablet PC, a notebook computer, or a personal digital assistance (PDA) through the wired or wireless network.

The input device 300 may output an electrical signal according to a user's manipulation and deliver the output electrical signal to the controller 210. Thus, the controller 210 may receive various commands associated with control of the ultrasonic imaging apparatus 272 from the user. The input device 300 may include, for example, at least one of a keyboard, a mouse, a track ball, a touchscreen, a touch pad, a paddle, various levers, a handle, a joystick, and various other input means.

The display 400 may display the generated or corrected ultrasonic image to a user. The display 400 may be implemented with a plasma display panel (PDP), a light emitting diode (LED), a liquid crystal display (LCD), or the like. Alternatively, the display 400 may use a three-dimensional display that may represent a stereoscopic image. The display 400 may include a touchscreen device. When the display 400 includes the touchscreen device, the display 400 may perform a function of the input device 300. The touchscreen device may be implemented with a resistive touchscreen panel or a capacitive touchscreen panel. Alternatively, the touchscreen device may be implemented with a touchscreen panel using an ultrasonic wave or an infrared ray.

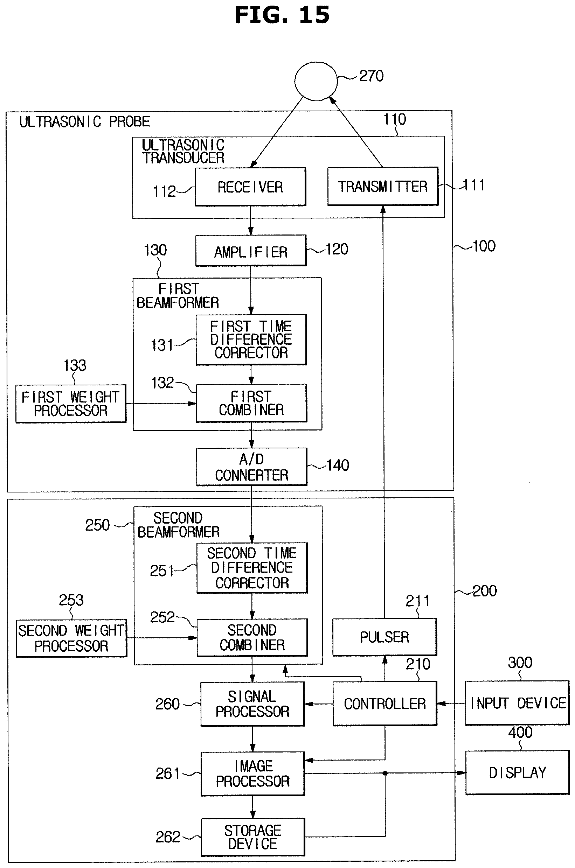

FIG. 15 is a block diagram showing an ultrasonic imaging apparatus according to an exemplary embodiment.

As shown in FIG. 15, the ultrasonic imaging apparatus 272 may include an ultrasonic probe 100 and a main body 200, and the ultrasonic probe 100 may include an amplifier 120 and the first beamformer 130. A function of the amplifier 120 of the ultrasonic probe 100 is the same as the function of the amplifier 220 of the above-described main body 200. As described above, the first beamformer 130 of the ultrasonic probe 100 may include a first time difference corrector 131 and a first combiner 132. Functions of the first beamformer 130, the first time difference corrector 131, and the first combiner 132 are the same as the functions of the first beamformer 230, the first time difference corrector 231, and the first combiner 232 of the above-described main body 200, respectively. For example, the ultrasonic probe 100 may further include a first weight processor 133 for calculating a first weight that is used by the first beamformer 130. In addition, the ultrasonic probe 100 may further include an A/D converter 140 for converting the first composite signal, which is an analog signal, into a digital signal. The A/D converter 140 may be provided in the main body 200, instead of the ultrasonic probe 100.

The main body 200 may include a second beamformer 250, a signal processor 260, an image processor 261, and a storage device 262.

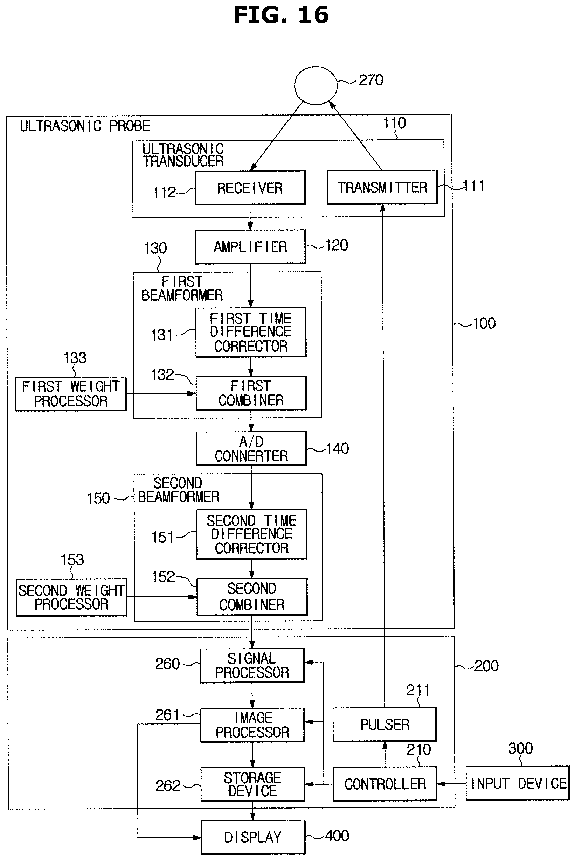

FIG. 16 is a block diagram showing an ultrasonic imaging apparatus according to an exemplary embodiment.

As shown in FIG. 16, the ultrasonic imaging apparatus 272 may include an ultrasonic probe 100 and a main body 200, and the ultrasonic probe 100 may include an amplifier 120, a first beamformer 130, a second beamformer 150, and a second weight processor 153. A function of the amplifier 120 of the ultrasonic probe 100 is the same as the function of the amplifier 220 of the above-described main body 200. As described above, the first beamformer 130 of the ultrasonic probe 100 may include a first time difference corrector 131 and a first combiner 132. Functions of the first beamformer 130, the first time difference corrector 131, and the first combiner 132 may be the same as the functions of the first beamformer 230, the first time difference corrector 231, and the first combiner 232 of the above-described main body 200, respectively. For example, the ultrasonic probe 100 may further include a first weight processor 133 for calculating a first weight that is used by the first beamformer 130. The ultrasonic probe 100 may further include an A/D converter 140 for converting the first composite signal, which is an analog signal, into a digital signal and delivering the digital-converted ultrasonic signal to the second beamformer 150. The second beamformer 150 of the ultrasonic probe 100 may include a second time difference corrector 151 and a second combiner 152. Functions of the second beamformer 150, the second time difference corrector 151, and the second combiner 152 may be the same as the functions of the second beamformer 250, the second time difference corrector 251, and the second combiner 252 of the above-described main body 200, respectively. The second beamformer 150 of the ultrasonic probe 100 may deliver the output second composite signal to the main body 200 through a connection cable 101 or the like. The second weight processor 153 of the ultrasonic probe 100 may determine a second weight that is varied depending to the first composite signal used by the second beamformer 250. A function of the second weight processor 153 of the ultrasonic probe 100 may be the same as the function of the second weight processor 253 of the main body 200.

The main body 200 may include a signal processor 260, an image processor 261, a storage device 262, and so on. The signal processor 260 may receive an ultrasonic signal from the second beamformer 150 of the ultrasonic probe 100.

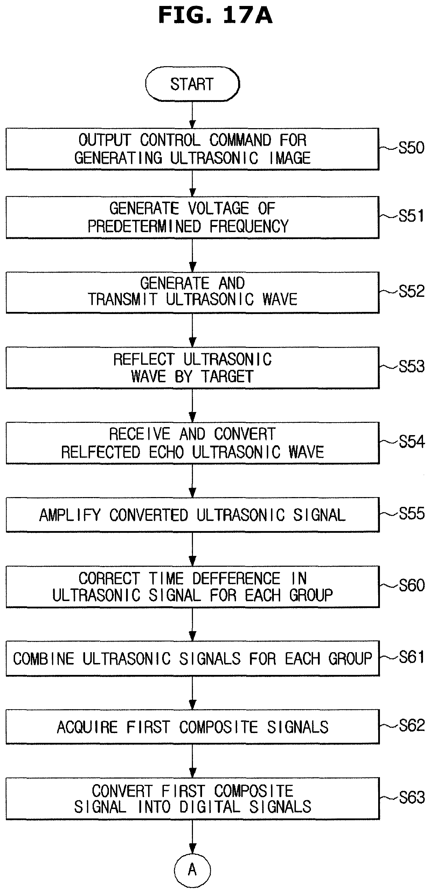

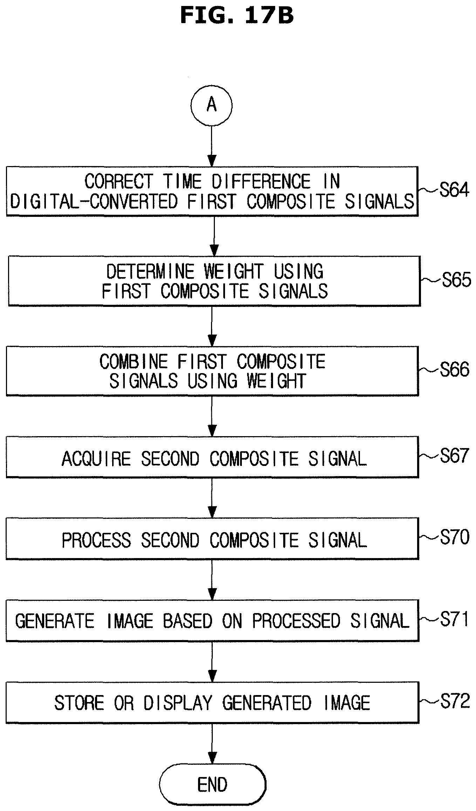

A control method described below with reference to FIGS. 17A and 17B according to an exemplary embodiment is performed by an ultrasonic imaging apparatus including a beamforming apparatus, which are described above with reference to FIGS. 1-16 and, thus, detailed repeated descriptions are omitted below.

As shown in FIG. 17A, a control command for generating an ultrasonic image according to a user's manipulation or the like is generated and output in operation s50. The controller 210 may generate and output the control command. A voltage of a predetermined frequency is generated in operation s51 and applied to the ultrasonic transducer 110a of the ultrasonic probe 100. The pulser 211 may generate a voltage of a predetermined frequency. The ultrasonic transducer 110a may generate an ultrasonic wave corresponding to the applied voltage and transmit the generated ultrasonic wave into the object 270 in operation s52. The transmitted ultrasonic wave may be reflected by a target inside the object 270 in operation s53, and the ultrasonic transducer 110a may receive an echo ultrasonic wave, which is the reflected ultrasonic wave, and convert the received echo ultrasonic wave into an ultrasonic signal which is an electrical signal, in operation s54. The ultrasonic signal may be amplified and delivered to the first beamformer, in operation s55. For example, the amplifier 120, 220 may amplify the ultrasonic signal and deliver the amplified signal to the first beamformer 10, 130, or 230.

The first beamformer may correct a time difference in the ultrasonic signal grouped into the groups, in operation s60, as described in detail above. The ultrasonic signals for each group may be combined in operation s61 and a plurality of first composite signals may be acquired in operation s62. For example, the first combiner 1, 12, 132, or 232 may combine the ultrasonic signals for each group. The plurality of first composite signals may be converted from analog format into digital signals in operation s63. For example, the A/D converters 31 through 33, 140, or 240 may convert the plurality of first composite signals into digital signals. The plurality of first composite signals may be delivered to the second beamformer 20, 150, or 250.

As shown in FIG. 17B, time differences between the plurality of first composite signals may be corrected in operation s64. For example, the second time difference corrector 21, 151, or 251 may correct the time differences between the plurality of first composite signals. When the time differences of the plurality of first composite signals are corrected, a weight may be determined using at least one of the plurality of first composite signals in operation s65, and the plurality of first composite signals may be combined using the calculated weight to acquire second composite signals in operations s66 and s67. The second weight processor 23, 153, or 253 may determine the weight and the second combiner 2, 22, 152, or 252 may combine the plurality of first composite signals using the calculated weight to acquire the second composite signal.

Various processing, such as filtering, may be performed on the second composite signal in operation s70, for example, by the signal processor 35 or 260. The ultrasonic image may be generated based on the processed signals in operation s71, for example, by the image processor 34 or 261. The generated ultrasonic image may be stored in the storage device 262 or displayed on the display 400 in operation s72. The above-described operations s50 to s72 may be repeatedly performed while ultrasonic imaging is performed.

According to the above-described beam forming apparatus, beam forming method, ultrasonic imaging apparatus, and ultrasonic probe, degradation of image quality may be minimized during a process of focusing a plurality of signals, thus acquiring a beam-formed image having good quality.

According to the above-described beam forming apparatus, beam forming method, ultrasonic imaging apparatus, and ultrasonic probe, an image having good quality may be acquired without sampling a signal with a high sampling frequency to form a beam, thus reducing an amount of data to be processed or transmitted in an apparatus.

According to the above-described beam forming apparatus, beam forming method, ultrasonic imaging apparatus, and ultrasonic probe, the amount of data to be processed or transmitted in an apparatus may be reduced, thus saving resources of the apparatus, for example, decreasing a capacity of a memory to be required for data processing or omitting various components.

According to the above-described ultrasonic imaging apparatus and ultrasonic probe, there is no need to implement an interpolation function for reducing data to be transmitted or processed in the ultrasonic imaging apparatus, thus improving simplicity in designing of the apparatus in addition to lightening and miniaturizing of the apparatus.

Furthermore, according to the above-described beam forming apparatus or beam forming method, it is also possible to obtain economic effects such as shortening manufacturing time of the apparatus requiring the beam forming process, such as an ultrasonic imaging apparatus or radar device, and/or saving manufacturing costs.

**Claims**

1.  A beam forming apparatus (269, 274) comprising:

    a first combiner (1, 12, 132, 232) configured to combine signals, which have been grouped into groups, and generate first composite signals; and
    a second combiner (2, 22, 152, 252) configured to combine the first composite signals,
    wherein the first combiner (1, 12, 132, 232) is further configured to combine the signals of each of the groups separately;
    wherein the second combiner (2, 22, 152, 252) is further configured to combine the first composite signals by applying a weight which varies depending on the first composite signals; and
    **characterized in that**
    at least one signal, which is grouped into one of the groups, is a same signal, which is grouped into another one of the groups.

2.  The beam forming apparatus (269, 274) of claim 1, further comprising:

    a first time difference corrector (11, 131, 231) configured to correct time differences between the signals of a respective group, separately for each group; and
    optionally a second time difference corrector (21, 151, 251) configured to correct time differences between the first composite signals.

3.  The beam forming apparatus (269, 274) of one of claims 1 to 2, wherein the first combiner (1, 12, 132, 232) is configured to combine the signals in one of the groups using a predefined weight or a weight determined based on at least one of the signals.

4.  The beam forming apparatus (269, 274) of one of claims 1 to 3, wherein the signals in the groups are analog signals, and the apparatus (269, 274) further comprises:

    an analog-to-digital converter (5, 31-33) configured to convert the signals in the groups into digital signals; or
    wherein the first composite signals are analog signals and the apparatus (269, 274) further comprises:
    an analog-to-digital converter (5, 31-33) configured to convert the first composite signals into digital signals.

5.  The beam forming apparatus (269, 274) of one of claims 1 to 4, further comprising:
    a weight processor (3, 13, 23, 133, 153, 233, 253) configured to determine the weight applied by the second combiner (2, 22, 152, 252) based on the first composite signals.

6.  The beam forming apparatus (269, 274) of one of claims 1 to 5, wherein a number of the first composite signals is less than a number of the signals grouped into the groups and input to the first combiner (1, 12, 132, 232).

7.  A beam forming method comprising:

    outputting (S40) signals grouped in groups;
    combining (S42) the signals in the groups separately for each of the groups to generate first composite signals; and

combining the first composite signals by applying a weight which varies depending on the first composite signals to generate a second composite signal; and
**characterized in that**
at least one signal grouped into one of the groups is a same signal which is grouped into another one of the groups.

8. The beam forming method of claim 7, further comprising:

correcting (S41) time differences between the signals of a respective group, individually for each group; and
optionally correcting (S45) time differences between the first composite signals.

9. The beam forming method of one of claims 7 to 8, wherein the combining the signals comprises:
combining the signals in one of the groups using a predefined weight or a weight determined based on at least one of the signals.

10. The beam forming method of one of claims 7 to 9, wherein the signals in the groups are analog signals, and the method further comprises:

converting the signals in the groups into digital signals; or
wherein the first composite signals are analog signals, and the method further comprises:
converting (S44) the first composite signals into digital signals.

11. The beam forming method of one of claims 7 to 10, wherein the combining the first composite signals comprises:

calculating (S46) a weight based on the first composite signals, and
applying the weight to the first composite signals.

12. The beam forming method of one of claims 7 to 11, wherein a number of the first composite signals is less than a number of the signals grouped into the groups.

**Patentansprüche**

1. Strahlformungsvorrichtung (269, 274), umfassend:

einen ersten Kombinierer (1, 12, 132, 232), der konfiguriert ist zum Kombinieren von Signalen, die zu Gruppen gruppiert wurden, und zum Erzeugen von ersten zusammengesetzten Signalen, und
einen zweiten Kombinierer (2, 22, 152, 252), der konfiguriert ist zum Kombinieren der ersten zusammengesetzten Signale,
wobei der erste Kombinierer (1, 12, 132, 232) weiterhin konfiguriert ist zum Kombinieren der Signale jeder der Gruppen jeweils separat,
wobei der zweite Kombinierer (2, 22, 152, 252) weiterhin konfiguriert ist zum Kombinieren der ersten zusammengesetzten Signale durch das Anwenden eines Gewichts, das in Abhängigkeit von den ersten zusammengesetzten Signalen variiert,
**dadurch gekennzeichnet, dass**
wenigstens ein Signal, das in eine der Gruppen gruppiert wurde, ein gleiches Signal, das in eine andere der Gruppen gruppiert wurde, ist.

2. Strahlformungsvorrichtung (269, 274) nach Anspruch 1, das weiterhin umfasst:

einen ersten Zeitdifferenzkorrigierer (11, 131, 231), der konfiguriert ist zum Korrigieren von Zeitdifferenzen zwischen den Signalen einer entsprechenden Gruppe jeweils separat für jede Gruppe, und
optional einen zweiten Zeitdifferenzkorrigierer (21, 151, 251), der konfiguriert ist zum Korrigieren von Zeitdifferenzen zwischen den ersten zusammengesetzten Signalen.

3. Strahlformungsvorrichtung (269, 274) nach Anspruch 1 oder 2, wobei der erste Kombinierer (1, 12, 132, 232) konfiguriert ist zum Kombinieren der Signale in einer der Gruppen unter Verwendung eines vordefinierten Gewichts oder eines basierend auf wenigstens einem der Signale bestimmten Gewichts.

**4.** Strahlformungsvorrichtung (269, 274) nach einem der Ansprüche 1 bis 3, wobei die Signale in den Gruppen analoge Signale sind und die Vorrichtung (269, 274) weiterhin umfasst:

einen Analog-zu-Digital-Wandler (5, 31-33), der konfiguriert ist zum Wandeln der Signale in den Gruppen zu digitalen Signalen, oder
wobei die ersten zusammengesetzten Signale analoge Signale sind und die Vorrichtung (269, 274) weiterhin umfasst:
einen Analog-zu-Digital-Wandler (5, 31-33), der konfiguriert ist zum Wandeln der ersten zusammengesetzten Signale zu digitalen Signalen.

**5.** Strahlformungsvorrichtung (269, 274) nach einem der Ansprüche 1 bis 4, die weiterhin umfasst:
einen Gewichtungsprozessor (3, 13, 23, 133, 153, 233, 253), der konfiguriert ist zum Bestimmen des durch den zweiten Kombinierer (2, 22, 152, 252) angewendeten Gewichts basierend auf den ersten zusammengesetzten Signalen.

**6.** Strahlformungsvorrichtung (269, 274) nach einem der Ansprüche 1 bis 5, wobei die Anzahl der ersten zusammengesetzten Signale kleiner als die Anzahl der in die Gruppen gruppierten und in den ersten Kombinierer (1, 12, 132, 232) eingegebenen Signale ist.

**7.** Strahlformungsverfahren, umfassend:

Ausgeben (S40) von in Gruppen gruppierten Signalen,
Kombinieren (S42) der Signale in den Gruppen jeweils separat für jede der Gruppen, um erste zusammengesetzte Signale zu erzeugen, und
Kombinieren der ersten zusammengesetzten Signale durch das Anwenden eines Gewichts, das in Abhängigkeit von den ersten zusammengesetzten Signalen variiert, um ein zweites zusammengesetztes Signal zu erzeugen,
**dadurch gekennzeichnet, dass**
wenigstens ein Signal, das in eine der Gruppen gruppiert wurde, ein gleiches Signal, das in eine andere der Gruppen gruppiert wurde, ist.

**8.** Strahlformungsverfahren nach Anspruch 7, das weiterhin umfasst:

Korrigieren (S41) von Zeitdifferenzen zwischen den Signalen einer entsprechenden Gruppe jeweils individuell für jede Gruppe, und
optionales Korrigieren (S45) von Zeitdifferenzen zwischen den ersten zusammengesetzten Signalen.

**9.** Strahlformungsverfahren nach Anspruch 7 oder 8, wobei das Kombinieren der Signale umfasst:
Kombinieren der Signale in einer der Gruppen unter Verwendung eines vordefinierten Gewichts oder eines basierend auf wenigstens einem der Signale bestimmten Gewichts.

**10.** Strahlformungsverfahren nach einem der Ansprüche 7 bis 9, wobei die Signale in den Gruppen analoge Signale sind und das Verfahren weiterhin umfasst:

Wandeln der Signale in den Gruppen zu digitalen Signalen, oder
wobei die ersten zusammengesetzten Signale analoge Signale sind und das Verfahren weiterhin umfasst:
Wandeln (S44) der ersten zusammengesetzten Signale zu digitalen Signalen.

**11.** Strahlformungsverfahren nach einem der Ansprüche 7 bis 10, wobei das Kombinieren der ersten zusammengesetzten Signale umfasst:

Berechnen (S46) eines Gewichts basierend auf den ersten zusammengesetzten Signalen, und
Anwenden des Gewichts auf die ersten zusammengesetzten Signale.

**12.** Strahlformungsverfahren nach einem der Ansprüche 7 bis 11, wobei die Anzahl der ersten zusammengesetzten Signale kleiner als die Anzahl der in die Gruppen gruppierten Signale ist.

**Revendications**

1. Appareil de formation de faisceau (269, 274) comprenant:

   un premier combineur (1, 12, 132, 232) configuré pour combiner des signaux, qui ont été regroupés, et générer les premiers signaux composites; et
   un second combineur (2, 22, 152, 252) configuré pour combiner les premiers signaux composites,
   dans lequel le premier combineur (1, 12, 132, 232) est en outre configuré pour combiner les signaux de chacun des groupes séparément;
   dans lequel le second combineur (2, 22, 152, 252) est en outre configuré pour combiner les premiers signaux composites en appliquant un poids qui varie en fonction des premiers signaux composites; et
   **caractérisé en ce que**
   au moins un signal, qui est regroupé dans l'un des groupes, est un même signal, qui est regroupé dans un autre desdits groupes.

2. Appareil de formation de faisceau (269, 274) selon la revendication 1, comprenant en outre:

   un premier correcteur de différence de temps (11, 131, 231) configuré pour corriger les différences de temps entre les signaux d'un groupe respectif, séparément pour chaque groupe; et
   éventuellement un second correcteur de différence de temps (21, 151, 251) configuré pour corriger les différences de temps entre les premiers signaux composites.

3. Appareil de formation de faisceau (269, 274) selon l'une des revendications 1 à 2, dans lequel le premier combineur (1, 12, 132, 232) est configuré pour combiner les signaux dans l'un des groupes en utilisant un poids prédéfini ou un poids déterminé sur la base d'au moins l'un des signaux.

4. Appareil de formation de faisceau (269, 274) selon l'une des revendications 1 à 3, dans lequel les signaux des groupes sont des signaux analogiques, et l'appareil (269, 274) comprend en outre:

   un convertisseur analogique-numérique (5, 31-33) configuré pour convertir les signaux des groupes en signaux numériques; ou
   dans lequel les premiers signaux composites sont des signaux analogiques et l'appareil (269, 274) comprend en outre:
   un convertisseur analogique-numérique (5, 31-33) configuré pour convertir les premiers signaux composites en signaux numériques.

5. Appareil de formation de faisceau (269, 274) selon l'une des revendications 1 à 4, comprenant en outre:
   un processeur de poids (3, 13, 23, 133, 153, 233, 253) configuré pour déterminer le poids appliqué par le second combineur (2, 22, 152, 252) sur la base des premiers signaux composites.

6. Appareil de formation de faisceau (269, 274) selon l'une des revendications 1 à 5, dans lequel un nombre de premiers signaux composites est inférieur à un nombre de signaux groupés dans les groupes et entrés dans le premier combineur (1, 12, 132, 232).

7. Procédé de formation de faisceau comprenant:

   la production (S40) de signaux regroupés;
   la combinaison (S42) des signaux des groupes séparément pour chacun des groupes afin de générer les premiers signaux composites; et
   la combinaison des premiers signaux composites en appliquant un poids qui varie en fonction des premiers signaux composites pour générer un second signal composite; et
   **caractérisé en ce que**
   au moins un signal regroupé dans l'un des groupes est un même signal qui est regroupé dans un autre desdits groupes.

8. Procédé de formation de faisceau selon la revendication 7, comprenant en outre:

   la correction (S41) des différences de temps entre les signaux d'un groupe respectif, individuellement pour chaque groupe; et
la correction éventuelle (S45) les différences de temps entre les premiers signaux composites.

**9.** Procédé de formation de faisceau de l'une des revendications 7 à 8, dans laquelle la combinaison des signaux comprend:
la combinaison des signaux dans un des groupes en utilisant un poids prédéfini ou un poids déterminé en fonction d'au moins un des signaux.

**10.** Procédé de formation de faisceau de l'une des revendications 7 à 9, dans laquelle les signaux dans les groupes sont des signaux analogiques, et le procédé comprend en outre:

la conversion des signaux dans les groupes en signaux numériques; ou
dans lequel les premiers signaux composites sont des signaux analogiques, et le procédé comprend en outre:
la conversion (S44) des premiers signaux composites en signaux numériques.

**11.** Procédé de formation de faisceau de l'une des revendications 7 à 10, dans laquelle la combinaison des premiers signaux composites comprend:

le calcul (S46) d'un poids fondé sur les premiers signaux composites, et
l'application du poids aux premiers signaux composites.

**12.** Procédé de formation de faisceau selon l'une des revendications 7 à 11, dans lequel un nombre de premiers signaux composites est inférieur à un nombre de signaux regroupés dans les groupes.

# FIG. 1

$$M \geq N \geq P$$

**FIG. 2**

# FIG. 3

SIGNAL
OUTPUT UNIT

4

M
SIGNALS (A)

A/D
CONVERTER

5

N
SIGNALS (D)

FIRST
BEAMFORMER

10

**FIG. 4**

# FIG. 5

# FIG. 6

# FIG. 7

# FIG. 8

# FIG. 9

START

OUTPUT ANALOG SIGNALS IN GROUPS — S40

CORRECT TIME DIFFERENCES IN
SIGNALS WITHIN GROUPS — S41

COMBINE SIGNALS FOR EACH GROUP — S42

ACQUIRE FIRST COMPOSITE SIGNALS — S43

CONVERT FIRST COMPOSITE SIGNALS
INTO DIGITAL SIGNALS — S44

CORRECT TIME DIFFERENCE IN
FIRST COMPOSITE SIGNALS — S45

DETERMINE WEIGHT — S46

COMBINE FIRST COMPOSITE
SIGNALS BASED ON DETERMINED WEIGHT — S47

ACQUIRE SECOND COMPOSITE SIGNAL — S48

END

# FIG. 10

# FIG. 11

270

**ULTRASONIC PROBE**

ULTRASONIC
TRANSDUCER

112 RECEIVER    TRANSMITTER 111    110    100

211

**MAIN BODY**

220 AMPLIFIER    PULSER    200

**FIRST BEAMFORMER**

231 FIRST TIME DIFFERENCE CORRECTOR

233

210    300

232 FIRST COMBINER    FIRST WEIGHT PROCESSOR    CONTROLLER    INPUT DEVICE

230    240 A/D CONNERTER

**SECOND BEAMFORMER**

251 SECOND TIME DIFFERENCE CORRECTOR

252 SECOND COMBINER    SECOND WEIGHT PROCESSOR 253

250    260 SIGNAL PROCESSOR

261 IMAGE PROCESSOR    400    DISPLAY

262 STORAGE DEVICE

# FIG. 12

# FIG. 13

105

110a

# FIG. 14

# FIG. 15

# FIG. 16

## FIG. 17A

```
                    START

        OUTPUT CONTROL COMMAND FOR          S50
        GENERATING ULTRASONIC IMAGE

            GENERATE VOLTAGE OF             S51
           PREDETERMINED FREQUENCY

              GENERATE AND                  S52
          TRANSMIT ULTRASONIC WAVE

            REFLECT ULTRASONIC              S53
             WAVE BY TARGET

            RECEIVE AND CONVERT             S54
         RELFECTED ECHO ULTRASONIC WAVE

       AMPLIFY CONVERTED ULTRASONIC SIGNAL  S55

          CORRECT TIME DEFFERENCE IN        S60
        ULTRASONIC SIGNAL FOR EACH GROUP

     COMBINE ULTRASONIC SIGNALS FOR EACH GROUP   S61

        ACQUIRE FIRST COMPOSITE SIGNALS     S62

          CONVERT FIRST COMPOSITE           S63
         SIGNAL INTO DIGITAL SIGNALS

                     A
```

34

## FIG. 17B

A

CORRECT TIME DIFFERENCE IN
DIGITAL-CONVERTED FIRST COMPOSITE SIGNALS — S64

DETERMINE WEIGHT USING
FIRST COMPOSITE SIGNALS — S65

COMBINE FIRST COMPOSITE
SIGNALS USING WEIGHT — S66

ACQUIRE SECOND COMPOSITE SIGNAL — S67

PROCESS SECOND COMPOSITE SIGNAL — S70

GENERATE IMAGE BASED ON PROCESSED SIGNAL — S71

STORE OR DISPLAY GENERATED IMAGE — S72

END

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012035723 A1
- WO 0217296 A1
- WO 2010137453 A1